# EUROPEAN PATENT SPECIFICATION

(11) **EP 2 675 397 B1**
(45) Date of publication and mention of the grant of the patent: **23.09.2015**
(21) Application number: 12713805.5
(22) Date of filing: 14.02.2012
(51) Int. Cl.: A61F 2/24, A61B 17/34, A61M 25/00

(54) **PERCUTANEOUS POSITIONING DEVICE**
PERKUTANE POSITIONIERUNGSVORRICHTUNG
DISPOSITIF DE POSITIONNEMENT PERCUTANÉ

(30) Priority: 15.02.2011 US 201161442888 P; 25.10.2011 US 201161551010 P
(43) Date of publication of application: 25.12.2013
(73) Proprietor: Medivalve Ltd., 20174 Misgav (IL)
(72) Inventor: KLEIN, Assaf, 38857 Hama'apil (IL); HEFER, Gil, 17906 Shimshit (IL)
(74) Representative: Hocking, Adrian Niall
(86) International application number: PCT/US2012/024942
(87) International publication number: WO 2012/112469

(56) References cited:
- EP-A1- 1 935 378
- WO-A2-2005/039428
- US-A1- 2006 195 134
- US-A1- 2007 038 293

## Description

### FIELD OF THE INVENTION

The present invention generally relates to percutaneous (or transcatheter) procedures of implanting a device through an opening. More specifically, it relates to a method for the placement of a valve in a heart in a more accurate way that may reduce the risks associated with inaccurate placement of such valves.

### BACKGROUND OF THE INVENTION

Many devices are known for percutaneous delivery of valves and other cardiovascular devices, but a need exists for proper positioning of such devices, such as but not limited to, positioning of artificial heart valves. A similar positioning device is disclosed in WO 2005/039428 A2.

### SUMMARY OF THE INVENTION

The present invention as defined in claim 1 seeks to provide novel percutaneous positioning devices.

In one non-limiting embodiment, the present invention provides novel structure for the placement of trans-apically or percutaneously implanted heart valves, e.g., the aortic valve. A flexible element of a device is deformed and moved towards and pushed against the structure in the vasculature where it is desired to place a prosthesis or other devices. The operator sees the deformation on imaging equipment and can position the device accordingly in its proper position.

The device may be intended for, but not limited to, the cardiovascular vessels and may be intended for, but not limited to, marking a specific location or structural tissue, locating, anchoring or stabilizing devices, protecting a vessel, or snaring, extricating, or otherwise moving another device or implant. The deployment or expansion of the apparatus may be from a small outer diameter (e.g., 0.3-10 mm) to a stable and much larger diameter (e.g., 1-50 mm).

The present invention includes also a delivery mechanism that includes an outer tube which keeps the prosthesis in its crimped state until it is positioned across the native valve, e.g., a prosthesis valve that is kept in a crimped state until a constraining sheath is pulled back. The delivery mechanism may include a balloon which is located underneath the crimped valve prosthesis and which assists the device to reach its final deployed configuration.

### BRIEF DESCRIPTION OF THE DRAWINGS

The present invention will be understood and appreciated more fully from the following detailed description taken in conjunction with the drawings in which:
Fig. 1 is a simplified illustration of a percutaneous positioning device, constructed and operative in accordance with a non-limiting embodiment of the present invention, showing a first deployment configuration, in which a distal member is folded compactly;
Figs. 2A-6C are simplified illustrations of the percutaneous positioning device of Fig. 1, wherein the distal member is deployed to a second deployment configuration, in which the distal member expands outwards and undergoes eversion, and then refolded in a reverse sequence back towards the first deployment configuration;
Figs. 6D and 6E are simplified illustrations of desirable and undesirable expanded orientations, respectively, of the loops of slender elements of the embodiment of Figs. 2A-6C;
Fig. 6F is a simplified illustration of a rotational orientation limiter that ensures the loops of the slender elements are rotationally orientated correctly with respect to the guidewire of delivery system;
Fig. 7 is a simplified illustration of a percutaneous positioning device, wherein a guidewire of the device passes through structure in a vasculature of a patient, e.g., a calcified aortic valve, and the guidewire passes into the left ventricle;
Fig. 8 is a simplified illustration of a distal end of the percutaneous positioning device of Fig. 7 expanded outwards to a second deployment configuration;
Fig. 9 is a simplified illustration of the distal end of the percutaneous positioning device of Fig. 8 being moved proximally against the structure in the vasculature of the patient;
Fig. 10 is a simplified illustration of compression of the distal end of the percutaneous positioning device of Fig. 9;
Figs. 11A, 11B, 12A, 12B and 12C are simplified illustrations of a valve prosthesis, and
Figs. 13A-13D are simplified illustrations of deployment of the valve prosthesis of Figs. 11A-12C across the native valve (valve annulus),

### DETAILED DESCRIPTION OF EXEMPLARY EMBODIMENTS

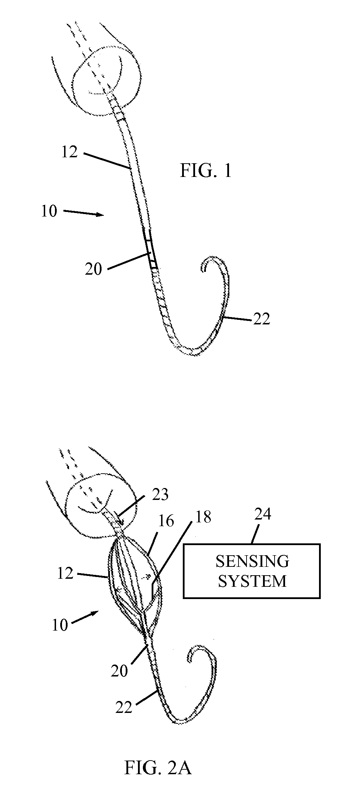

Reference is now made to Figs. 1-2A, which illustrate a percutaneous positioning device, constructed and operative in accordance with a non-limiting embodiment of the present invention.

The percutaneous positioning device includes a positioning member 10 that includes a flexible member 12, which may include one or more thin, elongate flexible elements 16 (e.g., struts, wires, strips and the like). Limiting members 18 (Fig. 2A) may be connected to one or more of the thin, elongate flexible elements 16, which limit the outward expansion of elements 16 during eversion thereof. The percutaneous positioning device may be made, without limitation, of stainless steel, shape memory materials or other suitably flexible and medically safe materials.

For example, in a preferred embodiment (although the invention is not limited to this embodiment), the percutaneous positioning device is delivered by a delivery system 22 (suitable guidewire or catheter system, well known in the art). An abutment member 20 located distal to member 12 for abutting against a portion of delivery system 22, such as a catheter or sheath, for example. Flexible member 12 is reversibly deformable between a first deployment configuration and a second deployment configuration. Fig. 1 illustrates flexible member 12 in the first deployment configuration, in which flexible member 12 is folded compactly. The percutaneous positioning device is shown delivered through the aortic valve, but the invention is not limited to any particular valve and may be used for the pulmonary valve, mitral valve, and tricuspid valve, as well as for delivering devices to other places in the body.

Figs. 2A-6C illustrate flexible member 12 in the second deployment configuration, in which flexible member 12 expands outwards, undergoes eversion and then is refolded in a reverse sequence back towards the first deployment configuration, as will be explained.

In Fig. 2A, the percutaneous positioning device is starting to be deployed. A distally-directed pushing force 23 moves flexible member 12 towards abutment member 20. When abutment member 20 abuts against delivery system 22, slender elements 16 start to bend outwards; thus, flexible member 12 starts to deform. In another option, the percutaneous positioning device is normally in its expanded position and is kept folded in an outer sheath or locking ring or other mechanism (not shown). For deployment, the locking mechanism would be removed and then the positioning device expands radially outwards (instead of expansion by axially directed forces in the embodiment of Figs. 2A-6C).

A sensing system 24, such as, but not limited to, an imaging system (e.g., fluoroscopy, MRI, etc.) is provided for sensing that flexible member 12 has been deformed.

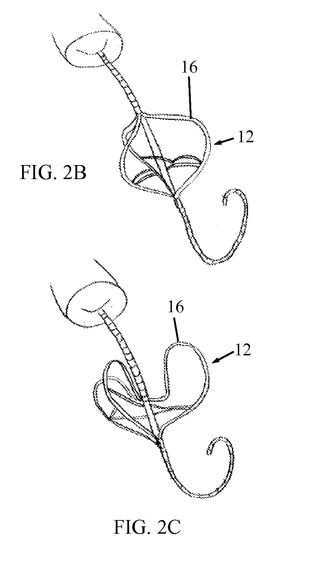

In Fig. 2B, the slender elements 16 continue to deform outwards. In Fig. 2C, a distally directed force of sufficient magnitude on flexible member 12 causes eversion of flexible member 12. The thin, elongate flexible elements 16 form loops after the eversion.

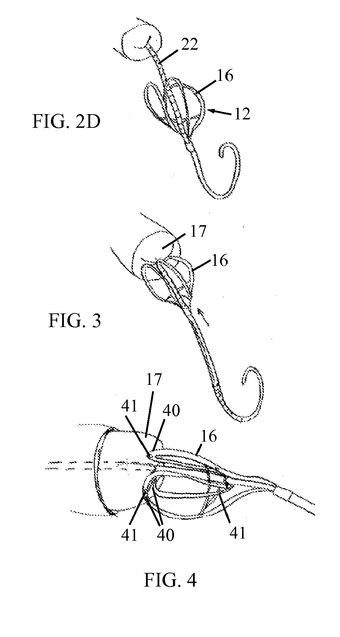

In Fig. 2D, flexible member 12 has completed eversion and reached the second deployment configuration, which is a stable working condition. If a particular application so requires, flexible elements 16 can be locked on delivery system 22 (e.g., guidewire) in a specific required condition.

In Fig. 3, the slender elements 16 are moved proximally against structure 17 of the vasculature of a patient, such as the natural valve. As also seen in Fig. 4, proximal portions of loops formed by the eversion of slender elements 16 define one or more vasculature abutment members 40 for abutting against structure 17. Markers 41 may be provided on abutment members 40 or other places on slender elements 16 for facilitating sensing when slender elements 16 have abutted against structure 17 (by using sensing system 24, not shown here). In this embodiment, as well as all the other embodiments of the invention, markers 41 may be placed either distally or proximally to spring-like portions of the device. Depending on their position, markers 41 may indicate the desired position when adjacent markers come closer together (e.g., such as in the case of a compression spring which becomes more compressed when squeezed together) or when adjacent markers go further apart (e.g., such as in the case of a tension spring which becomes pulled).

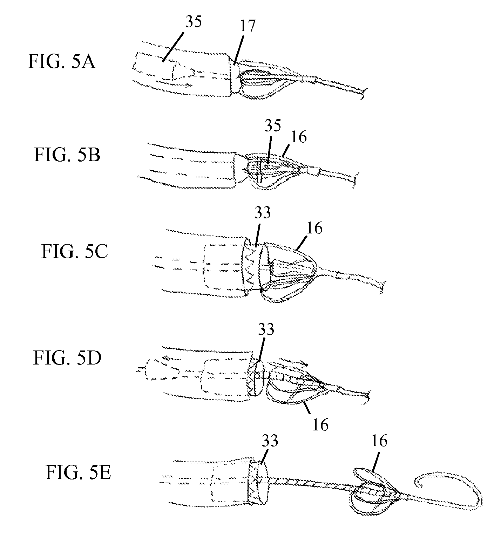

Figs. 5A-5E illustrates a cardiovascular prosthesis 33 (e.g., a replacement cardiac valve) being delivered to the structure 17 (e.g., over delivery system 22) and located at a predefined position with respect to flexible member 12. In Fig. 5A, a constraining sheath 35 is delivered over delivery system 22, and in Fig. 5B, passes through structure 17 (the native valve) and enters the space between slender elements 16. In Fig. 5C, prosthesis 33 is extracted from constraining sheath 35 and positioned in place of the native valve. In Fig. 5D, the constraining sheath 35 is retrieved in the proximal direction and slender elements 16 are moved distally away from prosthesis 33 to the position shown in Fig. 5E. In the position of Fig. 5E, abutment member 20 may "click" into a detent, catch or other structure in delivery system 22 so that member 20 is held in place during the refolding sequence. Alternatively, the device may be held in place by other methods, such as but not limited to, pulling backward into a catheter or by using an additional tube that holds the device in place.

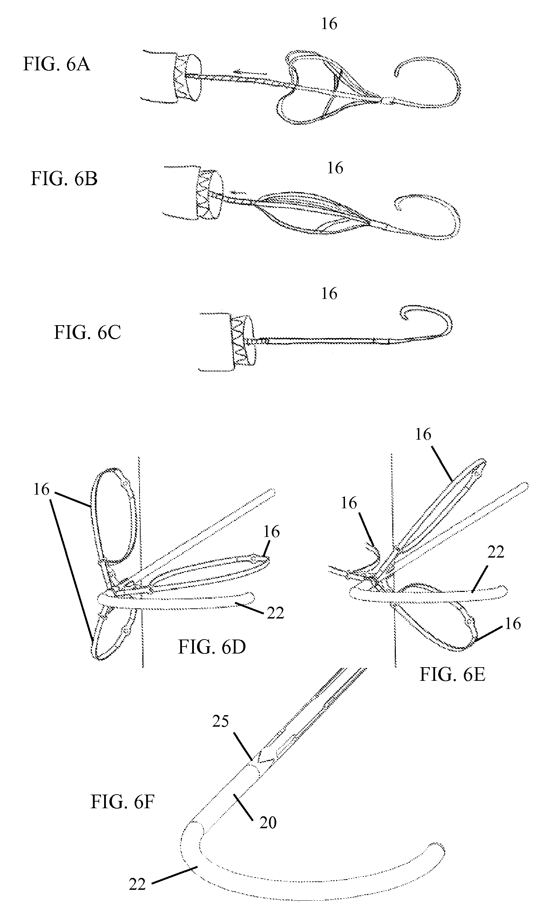

In Figs. 6A-6C, the device is refolded in a reverse sequence back towards the first deployment configuration.

It is noted that in one of the deployment configurations (e.g., the second one) the flexible member 12 is axially shorter and radially larger than in the other deployment configuration (the first one). Before the eversion, the most proximal part of the flexible member 12 is proximal to the vasculature abutment member 40, but after the eversion, the vasculature abutment member 40 becomes the most proximal portion of the flexible member 12.

Reference is now made to Figs. 6D-6F, which illustrate a further feature of the invention. In Fig. 6D, the desired expanded orientation of the loops of slender elements 16 is shown with respect to the guidewire of delivery system 22. The loops rest against the chamber wall (e.g., left ventricle) of the heart near the valve. In Fig. 6E, an undesirable expanded orientation of the loops is shown, wherein the device is turned so that one of the loops does not lie flat against the chamber wall.

Reference is now made to Fig. 6F. To avoid such undesirable orientations, a rotational orientation limiter 25 may be provided that ensures the loops of slender elements 16 are rotationally orientated correctly with respect to the guidewire of delivery system 22. The rotational orientation limiter 25, which may be part of abutment member 20, may include a male-female connection, such as but not limited to mating crowns, or any kind of protrusions or teeth that mesh with corresponding notches or grooves) with delivery system 22. This ensures the guidewire of delivery system 22 is always pressed against the chamber wall; the curved distal end of the guidewire is always directed away from the chamber wall (e.g., the left ventricle). The rotational orientation ensures that during expanding of the device no loop will be open in the direction of the chamber wall.

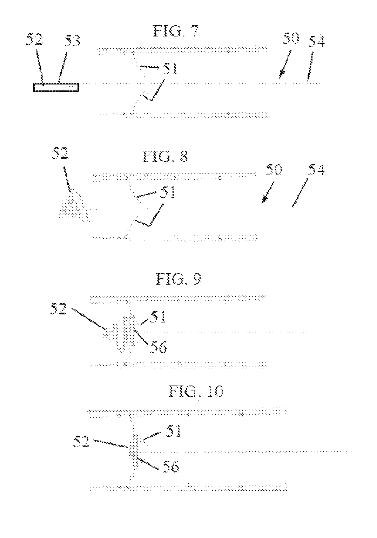

Reference is now made to Figs. 7-10, which illustrate a percutaneous positioning device, constructed and operative in accordance with another non-limiting embodiment of the present invention.

In Fig. 7, an elongate flexible member 50 (also called a positioning member 50, e.g., a guidewire) of the device passes through structure 51 in a vasculature of a patient, e.g., a calcified aortic valve, and the guidewire passes into the left ventricle. A distal member 52 of flexible member 50 may be initially compacted. For example, without limitation, an external sheath 53 may initially envelope the distal member 52 and is then removed by moving with a suitable guidewire. Alternatively, flexible member 50 could be initially shaped as a tube and initially kept in that shape with an internal or external holding mechanism. When the holding mechanism is moved or removed, the tube assumes a helical shape, for example. Alternatively, distal member 52 may be made of a shape memory material and expand outwards after a stress or temperature change or the like. In this manner, the distal member 52 can assume a shape, when expanded, which is larger in diameter than the opening through which it was entered. Without limitation, one configuration may be a helical spring shape with a pitch larger than 1 mm and less than 10 mm. As above, a proximal member 54 may be connected to delivery system 22, not shown here.

In Fig. 8, the distal member 52 expands outwards from the first deployment configuration to the second deployment configuration. In the second deployment configuration, distal member 52 includes a plurality of nested, resilient coils positioned distally, and the most proximal of the coils is a vasculature abutment member 56, which has the largest diameter of the coils. In Fig. 9, the vasculature abutment member 56 of distal member 52 is moved proximally against the structure 51 in the vasculature of the patient. The distal member 52 cannot pass back through the calcified valve. Fig. 10 illustrates compression of the distal member 52. The helical spring shape is compressed (pitch is decreased). As above, the operator can see the compressed state by imaging and thus know that the device has reached the annulus line.

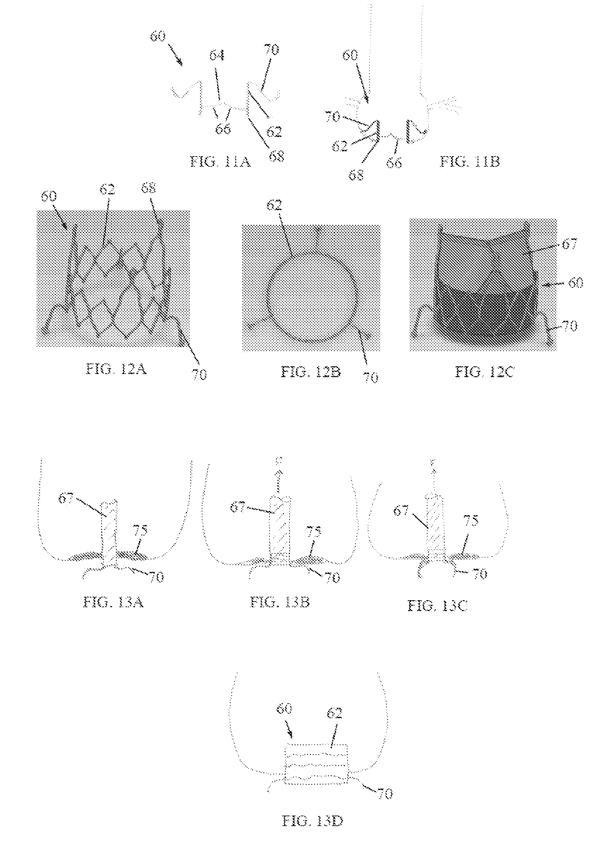

Reference is now made to Figs. 11A, 11B, 12A, 12B and 12C, which illustrate a valve prosthesis 60, including a mechanism for accurately positioning across the native calcified valve,

Valve prosthesis 60 includes a valve frame 62 and a valve 64 made of at least two leaflets 66. The frame 62 has a distal portion 68, which is shaped (e.g., folded or bent) to lean against the aortic root sinuses. This feature gives positive positioning and orientation as well as stability for the valve. Valve frame 62 of prosthesis 60 is initially disposed in a constraining sheath 67 (Fig. 12C). Valve frame 62 which has two or more flaps 70 that radially open when the constraining sheath 67 is removed.

Reference is now made to Figs. 13A-13D, which illustrate deployment of valve prosthesis 60 across the native valve (valve annulus) 75. Fig. 13A illustrates flaps 70 exposed below the valve annulus with the constraining sheath 67 pulled back a bit. Fig. 13B illustrates flaps 70 fully exposed and deployed radially. Flaps 70 are expanded below (that is, at the ventricular side) of valve 75 and act as movable markers for the delivery system while being pulled back by the operator. Flaps 70 abut the ventricular side of the calcified valve leaflets and deform in shape. This marks the ventricular margin of the calcified aortic valve to the operator for accurate positing of the valve prosthesis.

Fig. 13C illustrates the constraining sheath 67 of the delivery system pulled back (away from the valve - towards the aorta). Flaps 70 engage the native valve leaflets and deform in a way that indicates to the operator that this is the location of the valve annulus. Fig. 13D illustrates the final state after the entire valve prosthesis 60 is deployed.

It will be appreciated by persons skilled in the art that the present invention is not limited by what has been particularly shown and described hereinabove. Rather the scope of the present is defined by the appended claims.

## Claims

1. A system comprising:
a positioning member (10) comprising a flexible member (12) which is reversibly deformable between a first deployment configuration and a second deployment configuration and having a plurality of elongate flexible elements (16) each of which is attached to a limiting member (18); and
a delivery system (22) connected to said positioning member (10) for delivering said positioning member (10) in the first configuration to a location in the vasculature of the patient and deforming said flexible member (12), from the first configuration to the second configuration, wherein in deforming to the second configuration, the elongate flexible elements (16) undergo eversion limited by the limiting members (18) to form loops having vasculature abutment members (40) located on the flexible elongate elements (16) for abutting against a structure (17) in the vasculature of a patient.

2. The system according to claim I, wherein in one of said deployment configurations said flexible member (12) is axially shorter and radially larger than in the other deployment configuration.

3. The system according to claim I, further comprising a sensing system (24) for sensing that said flexible member (12) has been deformed and that said flexible member (12) has reached said structure (17).

4. The system according to claim 1, wherein before deforming, a most proximal part of said flexible member (12) is proximal to said vasculature abutment member (40), but after said deforming, said vasculature abutment member (40) becomes the most proximal portion of said flexible member (12).

5. The system according to claim 1, further comprising a cardiovascular prosthesis (33) or a catheter deliverable to said structure (17) and located at a predefined position with respect to said flexible member (12).

6. The system according to claim 5, wherein said cardiovascular prosthesis (33) comprises a replacement cardiac valve.

7. The system according to claim 5, wherein said cardiovascular prosthesis (33) or a catheter enters a space between said slender elements (16).

8. The system according to claim 5, wherein markers (41) are provided on said slender elements (16), and said cardiovascular prosthesis (33) or a catheter enters a space between said slender elements (16) that is more distal than said markers (41).

## Patentansprüche

1. System umfassend:
ein Positionierelement (10), umfassend ein flexibles Element (12), das reversibel verformbar zwischen einer ersten Aufstellungskonfiguration und einer zweiten Aufstellungskonfiguration ist und eine Mehrzahl von länglichen, flexiblen Elements (16) besitzt, von denen jedes an einem Begrenzungselement (18) befestigt ist; und
ein Fördersystem (22), das mit dem Positionierelement (10) zur Förderung des Positionierelements (10) in der ersten Konfiguration zu einem Ort in dem Gefäßsystem des Patienten und zum Verformen des flexiblen Elements (12) von der ersten Konfiguration zu der zweiten Konfiguration verbunden ist, wobei die länglichen, flexiblen Elemente (16) bei Verformung in die zweite Konfiguration eine Verdrehung durchmachen, die von den Begrenzungselementen (18) begrenzt wird, um Schleifen mit Gefäßsystem-Ankerelementen (40) zu bilden, die an den flexiblen, länglichen Elementen (16) zur Verankerung gegen eine Struktur (17) in dem Gefäßsystem eines Patienten angeordnet sind.

2. System nach Anspruch 1, wobei das flexible Element (12) in einer der Förderkonfigurationen axial kürzer und radial größer als in der anderen Förderkonfiguration ist.

3. System nach Anspruch 1, das weiterhin ein Erfassungssystem (24) zur Erfassung besitzt, dass das flexible Element (12) verformt wurde und das flexible Element (12) die Struktur (17) erreicht hat.

4. System nach Anspruch 1, wobei vor der Verformung ein am weitesten proximaler Teil des flexiblen Elements (12) proximal zu dem Gefäßsystem-Ankerelement (40) angeordnet ist, aber nach der Verformung das Gefäßsystem-Ankerelement (40) der am weitesten proximale Teil des flexiblen Elements (12) wird.

5. System nach Anspruch 1, weiterhin umfassend eine kardiovaskuläre Prothese (33) oder einen Katheter, der zu der Struktur (17) gefördert werden kann und an einer festgelegten Position in Bezug auf das flexible Element (12) angeordnet ist.

6. System nach Anspruch 5, wobei die kardiovaskuläre Prothese (33) eine Austausch-Herzklappe umfasst.

7. System nach Anspruch 5, wobei die kardiovaskuläre Prothese (33) oder ein Katheter in einen Raum zwischen den schmalen Elementen (16) eintritt.

8. System nach Anspruch 5, wobei Marker (41) an den schmalen Elementen (16) bereitgestellt werden, und die kardiovaskuläre Prothese (33) oder ein Katheter in einen Raum zwischen den schmalen Elementen (16) eintritt, die bzw. der weiter distal als die Marker (41) angeordnet ist.

## Revendications

1. Système comprenant :
un organe de positionnement (10) comprenant un organe souple (12) déformable de manière réversible, entre une première configuration de déploiement et une deuxième configuration de déploiement, et comportant une pluralité d'éléments souples allongés (16) parmi lesquels chacun est relié à un organe de limitation (18) ; et
un système de délivrance (22) relié audit organe de positionnement (10) pour délivrer ledit organe de positionnement (10) dans la première configuration à un endroit dans le système vasculaire du patient et pour déformer ledit organe souple (12), de la première configuration vers la deuxième configuration, sachant que pendant la déformation vers la deuxième configuration, les éléments souples allongés (16) sont soumis à une éversion limitée par les organes de limitation (18), pour former des boucles présentant des organes de butée vasculaire (40) situés sur les éléments souples allongés (16), destinés à buter contre une structure (17) dans le système vasculaire d'un patient.

2. Système selon la revendication 1, dans lequel, dans l'une des configurations de déploiement, ledit organe souple (12) est axialement plus court et radialement plus grand que dans l'autre configuration de déploiement.

3. Système selon la revendication 1, comprenant en outre un système de détection (24) capable de détecter sur ledit organe souple (12) a été déformé et que ledit organe souple (12) a atteint ladite structure (17).

4. Système selon la revendication 1, dans lequel, avant la déformation, une partie la plus proximale dudit organe souple (12) est proximale audit organe de butée vasculaire (40), mais après ladite déformation, ledit organe de butée vasculaire (40) devient la partie la plus proximale dudit organe souple (12).

5. Système selon la revendication 1, comprenant en outre une prothèse cardiovasculaire (33) ou un cathéter fourni à ladite structure (17) et situé à un emplacement prédéfini par rapport audit organe souple (12).

6. Système selon la revendication 5 , dans lequel ladite prothèse cardiovasculaire (33) comprend une valve cardiaque de remplacement.

7. Système selon la revendication 5, dans lequel ladite prothèse cardiovasculaire (33) ou un cathéter pénètre dans un espace entre lesdits éléments minces (16).

8. Système selon la revendication 5, dans lequel des marqueurs (41) sont prévus sur lesdits éléments minces (16), et ladite prothèse cardiovasculaire (33) ou un cathéter pénètre dans un espace entre lesdits éléments minces (16), lequel est plus distal que lesdits marqueurs (41).
